(19) Europäisches Patentamt
European Patent Office
Office européen des brevets

(11) **EP 2 514 361 A1**

(12) **EUROPEAN PATENT APPLICATION**

(43) Date of publication:
24.10.2012 Bulletin 2012/43

(51) Int Cl.:
*A61B 5/055* (2006.01) *G01R 33/465* (2006.01)

(21) Application number: 11163573.6

(22) Date of filing: 22.04.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: **Université Catholique de Louvain**
1348 Louvain-La-Neuve (BE)

(72) Inventors:
• **Gallez, Bernard**
1428 Lillois (BE)

• **Jordan, Bénédicte**
1930 Zaventem (BE)
• **Magat, Julie**
1050 Ixelles (BE)

(74) Representative: **Pecher, Nicolas et al**
Pecher & de Groote sprl
Centre Monnet
Avenue Jean Monnet, 1
1348 Louvain-la-Neuve (BE)

(54) **In vivo quantification of a variation of oxygenation in a tissue by using a magnetic resonance imaging technique**

(57) The invention relates to a device (200) and a method for quantifying a variation of oxygenation in a tissue (10) by using a magnetic resonance imaging technique. The variation of oxygenation in a tissue (10) can be quantified from a measured variation of a proton longitudinal relaxation rate $\Delta R1$ and from calibration data. The method of the invention is characterized in that the variation of proton longitudinal relaxation rate $\Delta R1$ that is used is a variation of proton longitudinal relaxation rate of lipids rather than a variation of proton longitudinal relaxation rate of water molecules.

Fig. 1

## Description

### Field of the invention

The invention relates to an in vivo method for quantifying a variation of oxygenation in a tissue. The invention also relates to a device able to communicate with a magnetic resonance imaging apparatus and to a use of the method of the invention.

### Description of prior art

There is a crucial need in clinical practice to evaluate the effect of a pathological status or a treatment on the oxygenation status of a tissue. As methods are lacking, there is great interest in developing non-invasive methods for mapping tissue oxygenation, tissue hypoxia, or variation of oxygenation in a tissue. The field of application of the invention includes oncology, cardiovascular diseases, diabetes and transplantation as illustrative examples. In the field of oncology, most solid tumors contain regions of acute and chronic hypoxia that can herald a negative clinical prognosis for cancer patient. Tumor hypoxia is indeed acknowledged as a major factor of resistance of solid tumors to radiation therapy, see for instance the article by M Hockel et al., published in J Natl Cancer Inst 2001, 93: 266-276, or the article by J von Pawel et al., published in J Clin Oncol 2000, 18: 1351-1359. This stimulated considerable efforts in defining and evaluating therapeutic approaches designed to overcome tumor hypoxia as source of resistance (Kaanders, 2002). To bridge the gap between occurrence of tumor hypoxia and clinical radiation practice, there is a need to predict variations of oxygenation in a tumor following a procedure aimed to change the oxygenation in said tumor. Increase in tumor oxygenation can be achieved by increasing the delivery of oxygen in the tumor (by inhalation of oxygen-enriched gas, vasoactive compounds or drugs shifting the dissociation curve of hemoglobin) or by decreasing the oxygen consumption by tumor cells. Then, it will become possible to adapt a tumor treatment depending on its capability of change of oxygenation after a procedure aimed to alleviate tumor hypoxia. On the other hand, other strategies try to starve the tumors by decreasing the tumor oxygenation (for instance by using antiangiogenic agents or vascular disrupting agents). There is therefore a need for developing accurate non invasive and quantitative in vivo methods for quantifying changes of oxygenation in tumors. In cardiovascular diseases, many pathological situations involve a local ischemia with important or vital consequences for the patients (for instance cerebral stroke, heart ischemia, peripheral ischemia). The ability of a technique to monitor the effect of a therapeutic intervention (for instance drug administration, surgery, changes in nutrition, physical exercise) on the level of oxygenation would be helpful to guide the practitioner in evaluating the effect of his suggested intervention or medication. This is also true for peripheral ischemia associated to diabetes (including, the problem of ischemia observed in the diabetic foot) and in transplantation, as the ability of a transplanted organ to reoxygenate after transplantation is a crucial factor for the success of this surgery.

In the past, magnetic resonance imaging (MRI) techniques have been used for evaluating changes of oxygenation in tissues. Several groups have monitored the tumor response to high-oxygen-content gases by MR sequences sensitive to the effective proton transverse relaxation time $T2^*$ to evaluate a level of saturation in oxygen of haemoglobin (G Karczmar, published in NMR Biomed 1994; 7, 3-11; S Robinson, published in Int J Radiat Oncol Biol Phys 1995; 33, 855-859). However, $T2^*$ mapping shows limitations in terms of quantitative aspects suggesting that such a method is qualitative in nature (see for instance the article by C Baudelet, published in Magn Reson Med 48, 980-986 (2002) or in Curr. Med. Imag. Reviews 2005; 1, 229-243, or Jordan in Magn. Reson Med 2006; 56, 637-643).

An alternative MRI technique for evaluating changes of oxygenation in tissues relies on an increase of a proton longitudinal relaxation rate R1 (or in an equivalent manner a decrease of proton longitudinal relaxation time T1) of water containing oxygen, due to paramagnetic properties of oxygen. A measured change in R1 is, in theory, proportional to a variation of oxygen concentration in a tissue (see for instance the article by J P B O'Connor published in Int J Radiation Oncology Biol Phys vol. 75, No 4, 1209-1215 (2009)). In this method, the measures of variation of R1 relate to variations of a global R1 that is a mean value for different types of molecules that are present in studied tissues. Most of these molecules are water molecules in mammal tissues. Hence, in this method, the measured proton longitudinal relaxation rates R1 are in a major part due to water molecules.

The technique proposed by J P B O'Connor lacks in sensitivity. Therefore, there is a need to provide a method for in vivo quantifying a variation of oxygenation in a tissue with a higher sensitivity.

### Summary of the invention

According to a first aspect, it is an object of the invention to provide a method for in vivo quantifying a variation of oxygenation in a tissue and that has a higher sensitivity than other known techniques. To this end, the method of the invention uses a magnetic imaging technique comprising a generation of a static magnetic induction $B_0$ and a generation of a sequence of radio-frequency pulses. The in vivo method of the invention comprises the steps of:

a. performing a first measurement of a proton longitudinal relaxation rate R1 with said magnetic resonance imaging technique;

b. optionally modifying an oxygen exposure of said tissue;

c. performing a second measurement of a proton longitudinal relaxation rate R1 with said magnetic resonance imaging technique;

d. determining a value of variation of said proton longitudinal relaxation rate, $\Delta$R1, from the measurements carried out in steps a. and c.;

e. providing calibration data relating values of variation of said proton longitudinal relaxation rate, $\Delta$R1, to values of variation of oxygenation;

f. determining a value of variation of oxygenation in said tissue by using the value of variation of said proton longitudinal relaxation rate determined in step d., $\Delta$R1, and the calibration data provided in step e..

The method of the invention is characterized in that said proton longitudinal relaxation rate R1 is a proton longitudinal relaxation rate R1 of lipids.

As stated previously, it is known that a variation of T1 (or R1=1/T1) is indicative of an oxygenation variation in a tissue. The inventors have found that isolating a variation of R1 of lipids leads to a higher sensitivity than considering a global variation of R1 for which a major part is due to water molecules. Hence, by evaluating a variation of R1 of lipids, one can have a method for quantifying a variation of oxygenation in a tissue that has a higher sensitivity with respect to other known techniques. The use of calibration data allows one to have a quantitative method. With respect to other methods, the method of the invention has other advantages. Compared to proton longitudinal relaxation rate of water molecules, proton longitudinal relaxation rate of lipid is less influenced by external factors such as blood flow. The method of the invention does not need the administration of any exogenous contrast agent such as a silicon-compound, as used for instance by Kodibagkar VD (published in NMR Biomed 2008; 21, 899-907). The method of the invention uses an endogenous signal of lipids and is an in vivo method.

Preferably, the method of the invention is characterized in that said sequence of radio-frequency pulses is able to remove a contribution of protons of water molecules to said proton longitudinal relaxation rate R1 of lipids.

The method of the invention is preferably characterized in that said sequence of radio-frequency pulses comprises at least one saturation pulse able to predominantly excite protons of water molecules.

Preferably, the method of the invention is characterized in that said sequence of radio-frequency pulses further comprises at least one initial inversion pulse, and at least one excitation pulse of flip angle $\alpha$. Preferably, said flip angle $\alpha$ has a value equal to or smaller than 30°.

Preferably, said at least one excitation pulse has a mean frequency that is shifted by a frequency shift below a resonance frequency of water molecules. Preferably, this frequency shift is comprised between 0.8 ppm and 1 ppm of said resonance frequency of water molecules. More preferably, said frequency shift is comprised between 3.3 ppm and 3.7 ppm of said resonance frequency of water molecules.

Preferably, the calibration data are obtained from an in vitro experimental calibration procedure where levels of oxygenation from said in vitro experimental calibration procedure are transposed into values of variation of oxygenation.

Preferably the optional step b. comprises an inhalation of an oxygen-enriched gas by a mammal body. More preferably, the optional step b. comprises a placement of a tourniquet that allows a stricture of a part of a mammal body.

According to a second aspect, the invention relates to a device able to communicate with an MRI apparatus, said device comprising:

i. control means for sending instructions to said magnetic resonance imaging apparatus;

ii. acquisition means for acquiring at least two values of a proton longitudinal relaxation rate R1 obtained from at least two measurements carried out with said magnetic resonance imaging apparatus on a tissue;

iii. means for determining a value of variation of said proton longitudinal relaxation rate, $\Delta$R1, from the at least two values of a proton longitudinal relaxation rate R1 obtained from the at least two measurements;

iv. means for providing calibration data relating values of variation of said proton longitudinal relaxation rate $\Delta$R1 to values of variation of oxygenation;

v. means for determining, by using the calibration data, a variation of oxygenation in said tissue corresponding to the value of variation of said proton longitudinal relaxation rate $\Delta$R1 determined by means.

The device of the invention is characterized in that said instructions sent by said control means to said magnetic resonance imaging apparatus are such that said magnetic resonance imaging apparatus is able to measure a proton longitudinal relaxation rate R1 of lipids.

According to a third aspect, the invention relates to the use of a method according to the invention for in vivo quantifying a variation of oxygenation in a tissue when said variation of oxygenation is induced by a drug.

**Short description of the drawings**

These and further aspects of the invention will be explained in greater detail by way of example and with reference to the accompanying drawings in which:

Fig.1       shows an example of a sequence of radio-frequency pulses and of gradients that can be used with the method of the invention;

Fig.2       shows a typical temporal evolution of a signal S that can be measured with an MRI apparatus when applying the method of the invention;

Fig.3       shows an example of calibration curve with other measurements;

Fig.4       shows another example of calibration curve with other measurements;

Fig.5       shows an embodiment of a device according to the invention in relation with an MRI apparatus;

Fig.6       shows an example of a sequence of radio-frequency pulses that can be used with the method of the invention when a proton longitudinal relaxation rate R1 of water molecules is wanted;

Fig.7       shows relative changes of R1 of lipids and water molecules in percent in a brain of a mouse before and after an inhalation of carbogen;

Fig.8       shows relative changes of R1 of lipids and water molecules in percent in a liver of a mouse before and after an inhalation of carbogen;

Fig.9(a)    shows, for a mouse tumor, variations of proton longitudinal relaxation time T1 of water molecules, when oxygenation is changed;

Fig.9(b)    shows, for a mouse tumor, variations of proton longitudinal relaxation time T1 of lipids, when oxygenation is changed;

Fig.9(c)    shows, for a mouse tumor, histograms of the number of pixels of an MRI image having given R1 values before and after a change of tumor oxygenation when said R1 is the one of water molecules;

Fig.9(d)    shows, for a mouse tumor, histograms of the number of pixels of an MRI image having given R1 values before and after a change of tumor oxygenation when said R1 is the one of lipids.

The figures are not drawn to scale. Generally, identical components are denoted by the same reference numerals in the figures.

**Detailed description of preferred embodiments**

In vivo MRI techniques are well known by the one skilled in the art. In MRI, a static magnetic induction $\mathbf{B}_0$ is first applied to a tissue 10 to be studied. This static magnetic induction is preferably applied by a superconducting magnet 60 and is typically strong: $\mathbf{B}_0$ typically has a value larger than 1 T. Here, we assume that this static magnetic induction $B_0$ is applied along a z-axis. Following the application of this static magnetic induction $B_0$, an equilibrium state takes place and, as a first approximation, one can consider that a macroscopic magnetization $\overline{M}$ is induced along the z-axis in the studied tissue 10 which means $\overline{M}=m_0z$. Then, a sequence of radio-frequency (RF) pulses is typically applied to modify this equilibrium state. This equilibrium state is disturbed via transmission of photons with appropriate energy (resonance phenomena). For a proton of energy $E = \hbar\omega_0$, a resonance condition is described by a Larmor angular frequency : $\omega_0 = \gamma B_0$, where $\hbar$ is a reduced Planck constant, $\gamma$ is a gyromagnetic ratio. If $B_0$ = 1 T, the Larmor frequency $f_0$ is approximately 42.6 MHz for protons. The generation of RF pulses is sometimes called an excitation phase. As an example, a sequence of RF pulses can start with a 180° radio-frequency inversion pulse that flips the magnetization $\overline{M}$ along a negative direction of the z-axis. It is known by the one skilled in the art that RF pulses force all individual spins to rotate in phase. This phenomenon is called phase coherence, and it explains why in nonequilibrium conditions the magnetization $\overline{M}$ can have a transverse (which means perpendicular to the z-axis) component $M_{trans}$. Preferably, the RF pulses are generated by sending alternating currents in two coils positioned along an x-axis and an *y*-axis that are perpendicular to said z-axis. Preferably, the x-axis is perpendicular to the y-axis. Such a configuration is known by the one skilled in the art as a quadrature transmitter. In reality, an MRI sequence is a combination of RF pulses and gradients (linear variations along a direction) to acquire data that allow one to build an image. Typically, a first gradient allows one to select a slide of a tissue 10. Preferably this first gradient is applied parallel to the z-axis. Then, a second gradient (perpendicular to the first gradient) and named phase gradient is typically applied. Finally, a third gradient (perpendicular to the first and second gradient) and named frequency gradient is typically applied. The names of second and third gradients originate from the fact that a frequency domain is generally used when dealing with MRI techniques. The gradients are typically applied by gradient coils.

When RF pulses are switched off, the studied tissue 10 returns to its equilibrium state. The transverse component of $\overline{M}$, $M_{trans}$, returns to zero, and the longitudinal component (which means the $\overline{M}$ component along the z-axis, $M_z$) tends to $M_0$ again. This return to equilibrium is named relaxation and can be decomposed in a longitudinal relaxation and a transverse relaxation. The longitudinal relaxation is governed by equation (Eq. 1):

$$M_z(t) = M_0(1 - \exp(-{}^t/_{T_1})) \text{ (Eq. 1)},$$

whereas the transverse relaxation is given by equation (Eq. 2):

$$M_{trans}(t) = M_{0,trans} \exp(-{}^t/_{T_2}) \text{ (Eq. 2)}.$$

In these equations, t stands for the time where t = 0 corresponds to a time where the RF pulses are switched off, $T_1$ is named proton longitudinal relaxation time, $T_2$ is named proton transverse relaxation time, and $M_{0,trans}$ is the value of the transverse component $M_{trans}$ at t = 0. The time evolutions described by equations (Eq. 1) and (Eq. 2) can be measured by receivers or receiving antennas.

For in vivo determining a variation of oxygenation in a tissue 10, the inventors propose to use an MRI technique comprising a generation of a static magnetic induction $B_0$ and a generation of a sequence of RF pulses applied to the tissue 10. The oxygenation of a sample refers to the concentration of oxygen that is dissolved in this sample. There are several ways to express the concentration of oxygen. The air in the atmosphere has a total pressure of 760 mmHg (1 atmosphere of pressure = 760mmHg = 101 kPa). Air is made up of 21 % oxygen. The pressure of oxygen (p02) of dry air under 1 atm is therefore 159 mmHg. The dissolution of gases in liquids follows the Henry's law and depends on the solubility of the gas in a particular solvent. When equilibrated with air under atmospheric pressure at 25°C, the oxygen dissolved in water is 0.0086 g/l or 0.27 mM ($10^{-3}$ M). The inventors have found that isolating a variation of $T_1$ of lipids leads to a higher sensitivity than considering a global variation of $T_1$ whose major part is due to water molecules. Hence, the method of the invention comprises the following steps. First, one has to perform a first measurement of a proton longitudinal relaxation rate R1 of lipids by using a MRI technique applied to the studied tissue 10. Various values of static magnetic induction $B_0$ and various types of sequences of RF pulses can be used but one needs to use a static magnetic induction $B_0$ and a sequence of RF pulses that allow one to measure R1 of lipids. After this first measurement, one can optionally modify the oxygen exposure of the studied tissue 10. This step is not necessary as some tissues 10 such as tumors are known to have a spontaneous changing level of oxygenation (RD Braun published in Am J Physiol 1999; 277, H551-568), and oxygenation may vary physiologically in different tissues as a result of physiology, physiological exercise or nutrition. The variation of oxygenation of the tissue 10 can also be induced by any type of modulation (physiological, physical, nutritional, drug-induced, gas-induced) in oxygenation. As an example, an oxygen exposure of a tissue can be modified by an inhalation of an oxygen-enriched gas. Carbogen is an example of an oxygen-enriched gas. Another possibility to modify the oxygenation of a tissue 10 is to apply a tourniquet that allows a stricture of a part of a mammal body.

Then, one has to perform a second measurement of a proton longitudinal relaxation rate R1 of lipids by using a MRI technique applied to the studied tissue 10. As for the first measurement, one needs to use a sequence of RF pulses that allows one to measure the R1 of lipids. One will preferably use for the second measurement of R1 of lipids the same conditions as the ones used for the first measurement. That means the same value and direction of application of $B_0$, as well as the same sequence of RF pulses. From the first and second measurements, one can deduce a variation of the proton longitudinal relaxation rate ΔR1 of lipids. Preferably, such a variation is obtained by subtracting to the proton longitudinal relaxation rate R1 determined in the second measurement, the value of R1 determined in the first measurement: ΔR1=R1 (second measurement) - R1 (first measurement). Calibration data are then provided that relate values of variation of said proton longitudinal relaxation rate, ΔR1, to variation of oxygenation. Finally, a variation of oxygenation in the studied tissue 10 is obtained by using the variation of said proton longitudinal relaxation rate ΔR1 of lipids from first and second measurements and said calibration data. Preferably, the calibration data form a calibration curve that associates a value of variation of oxygenation for each value of variation of proton longitudinal relaxation rate ΔR1 of lipids. Examples of calibration curves are given below.

As known by the one skilled in the art, several methods exist to measure a proton longitudinal relaxation rate R1 of lipids. As mammal tissues comprise a great amount of water molecules, one preferably uses a sequence of RF pulses that allow one to suppress a signal of water molecules (see for instance Haacke, in "Magnetic Resonance Imaging: Physical Principles and Sequence Design" 1999, John Willey & sons). Preferably, one can use saturation pulses 30. This approach uses the fact that fat (or lipids) and water molecules have different Larmor frequencies (or resonant

frequencies). When $B_0$ is perfectly homogeneous, a sufficiently narrow RF pulse (that we name an excitation and saturation pulse 30) can be used to tip either species into a transverse plane. If such a pulse is used to excite water molecules, then an excitation pulse 40 applied shortly after said saturation pulse 30 only tips a magnetization of lipids into a transverse plane. Since water molecules have just been excited, their longitudinal magnetization has not had time to regrow and there is no water component to tip into said transverse plane. Hence, the resulting signal measurement should be primarily from lipids. It is said that the saturation pulse 30 has saturated a signal from water. Another possibility known by the one skilled to the art to suppress a signal of water molecules is to use a "Tissue Nulling with Inversion Recovery" technique. Such a technique takes advantage of T1 differences of lipids and water molecules by using an inversion recovery sequence. By inverting the lipids and water molecules longitudinal magnetization and then collecting the data at the zero crossing of the signal from water molecules, said signal from water molecules will be cancelled independent of static field variations. One can also use a "multiple point water/lipid separation" method. Based on Chemical Shift Imaging (CSI) methods, it is possible to determine the relative signal contribution from water molecules and lipid. Modifications of the sequence of RF pulses are used to collect two or three images with different phase information that can be manipulated to find separate water and fat images, including Gradient Echo (GE) approach, single echo separation, spin echo (SE) approach, two-point separation, and three-point separation, as illustrative examples.

Preferably, when one uses saturation pulses 30 for removing signal from water molecules, the mean frequency of said saturation pulses is 500 MHz when $B_0$ = 11.7 T, and more preferably the saturation pulses 30 have a frequency bandwidth of 1 kHZ. The saturation pulses 30 are preferably applied during 5.4 ms.

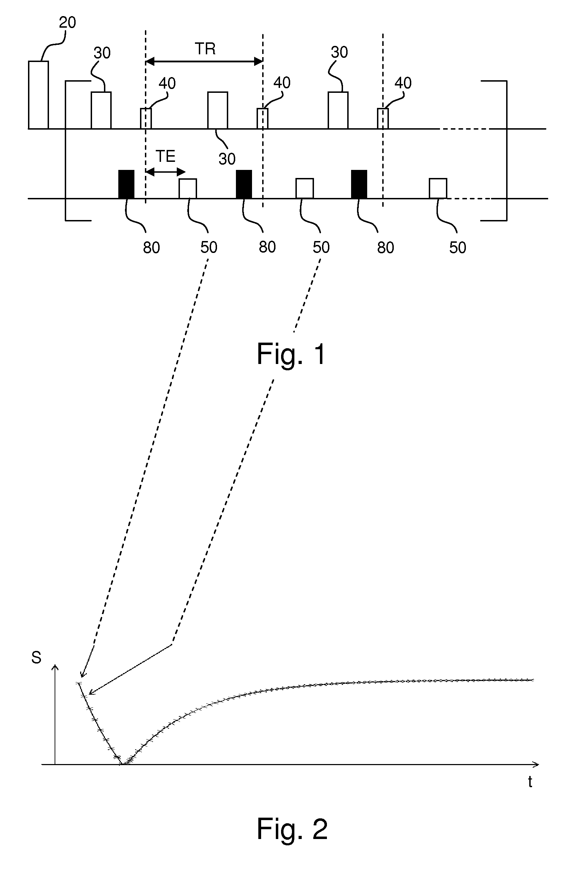

In a preferred embodiment, saturation pulses 30 used to suppress a contribution of water molecules are combined with excitation pulses 40 that extract T1 of lipids. As known by persons skilled in the art, several methods exist to measure T1 (as reviewed in : Gowland & Stevenson, in "Quantitative MRI of the brain", The longitudinal relaxation time, pp.111-141, Ed. P. Tofts, 2004): variants on the inversion recovery sequence, saturation recovery sequence, two points measurement method, presaturation method, stimulated echo sequence, spoiled Gradient-Echo, Look-Locker (LL) sequence, and chemical shift imaging (CSI) combined with inversion recovery and appropriate post-processing, can be used to map T1 of for instance lipids. An example of sequence of RF pulses that can be used with the method of the invention is illustrated in figure 1. This figure illustrates a Look-Locker (LL) sequence that presents the advantage of a short acquisition time adapted to in vivo studies. An initial inversion pulse 20 is applied. The inversion pulse 20 induces the magnetization $\overline{M}$ (that is initially parallel to the z-axis because of the application of the induction $B_0$) to flip of an angle of 180°. After, a saturation pulse 30 is applied and followed a gradient 80 (more precisely a frequency gradient). This gradient 80 allows one to induce a phase shift of a signal from water molecules (and so to better remove a signal from water molecules). After, an excitation pulse 40 is applied. An acquisition signal 50 is measured after a time TE of the excitation pulse 40. TE is usually named Echo Time. The saturation pulse 30 and the excitation pulse 40 are repeated each TR time and form what we name a sequence of RF pulses. The brackets in figure 1 represent the limits of the sequence. Typically, such a sequence lasts 1 min 20s which also corresponds to the total time of acquisition. TR is usually named Repetition Time. Preferably, one can use TR = 4 ms, TE = 1.2 ms.

In the Look-Locker (LL) sequence illustrated in figure 1, the excitation pulses 40 preferably have a constant, limited flip angle $\alpha$. A flip angle $\alpha$ of a RF pulse is known by the one skilled in the art. A flip angle lower than 90° decreases the amount of magnetization tipped into the transverse plane (plane perpendicular to the z-axis). The consequence of a low-flip angle $\alpha$ is a faster recovery of longitudinal magnetization that allows shorter TR/TE and decreases scan time. If $B_1$ is a magnitude of a magnetic induction of a RF excitation pulse 40 and $t_{ex}$ is the duration of the excitation pulse 40, then the flip angle $\alpha$ is given by equation (Eq. 3):

$$\alpha = \gamma B_1 t_{ex} \text{ (Eq. 3)},$$

where $\gamma$ is a gyromagnetic ratio which is a constant for a particular nucleus. As an example, $\gamma$ = 267,513 $10^6$ rad.s$^{-1}$T$^{-1}$ for a nucleus of hydrogen (or in an equivalent manner, $\gamma/2\pi$ = 42.57 MHz/T for a nucleus of hydrogen). The flip angle $\alpha$ represents a value of an angle between the z-axis and $\overline{M}$ after an application of an excitation pulse 40. Preferably the flip angle $\alpha$ of the excitation pulses 40 has a value equal to or lower than 30°. More preferably the flip angle $\alpha$ has a value of 5°.

The excitation pulses 40 have a frequency bandwidth and a mean frequency. Preferably, the mean frequency of the excitation pulses 40 is shifted by a frequency shift (also known as chemical shift) from a resonant frequency of water molecules. This allows one to predominantly excite other molecules than water molecules. In conventional MRI measurements, sequences of RF pulses are typically such that the excitation pulses are centered on a resonance frequency of water molecules. When $B_0$ = 11.7 T, the resonance frequency of water molecules is around 500 MHz. As the method of the invention aims at measuring a proton longitudinal relaxation rate R1 of lipids, one preferably uses a frequency shift for the mean frequency of the excitation pulses 40 such that they predominantly excite lipids (the article by M Kriat et al. published in Journal of Lipid Research, vol. 34, 1009 (1998) gives examples of resonance frequencies of lipids). For that, one has to know a frequency shift (or chemical shift) of lipids in a tissue 10 with respect to the resonance frequency of water molecules in the same tissue 10. This can be realized by identifying a lipid peak in a tissue 10 and by comparing the frequency corresponding to said lipid peak to the resonance frequency of water molecules. As illustrative examples, the inventors have found that a frequency shift (or chemical shift) equal to 0.9 ppm could be used to obtain T1 of lipids in experimental tumors. Similarly, a frequency shift of 3.5 ppm is preferably chosen for evaluating a T1 of lipids in liver. The notation ppm means part per million or $10^{-6}$. So, the frequency shift (or chemical shift) is expressed as a ratio between the frequency shift in Hz (between a lipid peak and a resonance frequency of water molecules) and a reference frequency. When using $B_0 = 11.7$ T, a typical reference frequency used by the one skilled in the art for defining frequency shifts in ppm (or chemical shifts) in MRI techniques is equal to 500 MHz, such a value corresponding to a reference frequency of water molecules. When applying a static magnetic induction equal to $B_0 = 11.7$ T, the frequency shift of the excitation pulses 40 is preferably equal to 450 Hz $\pm$ 10 % (which means a frequency shift between 0.8 and 1 ppm) for tumourous tissues and equal to 1750 Hz $\pm$ 10 % (which means a frequency shift between 3.3 and 3.7 ppm) for liver tissues. More preferably, the frequency shift of the excitation pulses 40 is equal to 0.9 ppm for tumourous tissues and equal to 3.5 ppm for liver tissues. As a summary, a preferred sequence of RF pulses for the method of the invention corresponding to an applied static magnetic induction equal to $B_0 = 11.7$ T, comprises :

- a inversion pulse 20 having a mean frequency of 500 MHz, a bandwidth equal to 5 kHz, applied during 15 ms,
- saturation pulses 30 having a mean frequency of 500 MHz, a bandwidth equal to 1 kHz, applied during 5.4 ms,
- excitation pulses 40 of flip angle $\alpha=5°$ having a mean frequency of 500 MHz - 450 Hz (for tumourous tissues) or 500 MHz - 1700 Hz (for liver tissues), a bandwidth equal to 5,4 kHz, and applied during 1 ms.

A bandwidth of 100 kHz is preferably chosen for the receiving antennas of the MRI apparatus 210. In clinical environments, the static magnetic induction $B_0$ typically has a smaller value than 11.7 T. Then, values of frequency shifts, mean frequency and bandwidth are adapted as known by the one skilled in the art.

Figure 2 shows a typical temporal evolution of a signal S that can be measured with an MRI apparatus when applying the method of the invention. S depends on the variation of the longitudinal component of the magnetization, $M_z$, when a sequence of RF pulses similar to the one of figure 1 is superposed to a static magnetic induction $B_0$ in a tissue 10. The different crosses of figure 2 correspond to different acquisition signals 50. The abscissa of figure 2 is the time, whereas the ordinate corresponds to said measured signal S (in arbitrary units). One can fit the different acquisition signals (crosses in figure 2) with a three parameters exponential curve to estimate a proton longitudinal relaxation time T1. This exponential curve is given by equation (Eq. 4):

$$S = \left| a - b \exp\left( {-t}/{T1} \right) \right| \ (\text{Eq. 4})$$

where a, b and the proton longitudinal relaxation time T1 are three parameters obtained from the fit. The one skilled in the art can use a least-square approximation for performing such a fit. Knowing T1, one can deduce a proton longitudinal relaxation rate R1 by using the relation R1=1/T1.

Commercial MRI apparatus are available with different possible sequences. Preferably, one can use a segmented Inversion-Recovery Fast Imaging with steady state Precession sequence on a Bruker Biospec 11.7 T system. This sequence allows one to perform a rapid monitoring of variations of R1 of lipids. Preferably, respiratory triggering is employed to measure the acquisition signals 50 during the expiration cycle to avoid motion artifacts.

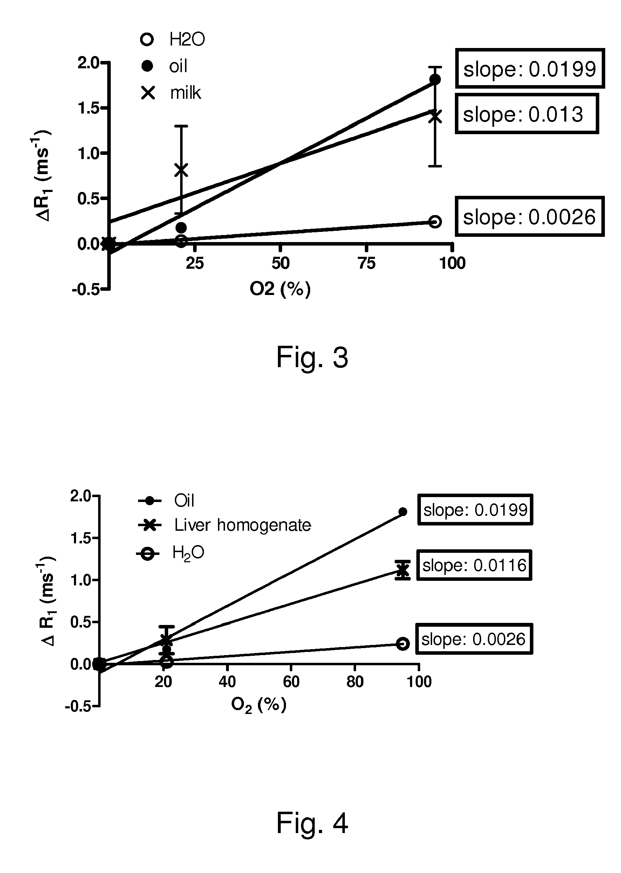

Different procedures are possible to obtain calibration data for the method of the invention. One can for instance simulate a relaxation of a magnetization of lipids having different levels of oxygenation to evaluate a variation of proton longitudinal relaxation rate $\Delta$R1 of lipids subjected to a static magnetic induction $B_0$ and a sequence of RF pulses. From such a calculation, one can obtain calibration data relating values of variation of $\Delta$R1 and values of variation of oxygenation. Preferably, one can obtain calibration data from an experimental calibration procedure in vitro. Experimental conditions (such as value and direction of application of $B_0$, sequence of RF pulses) used during such a calibration procedure are preferably the same as the ones used during the first and second measurements. Figure 3 and figure 4 show two examples of calibration curves. These figures also show that a change of oxygenation of lipids (such as oil) lead to larger change of proton longitudinal relaxation rates $\Delta$R1 with respect to water (H20). Hence, by considering the $\Delta$R1 of lipids, one has a method with a higher sensitivity for quantifying a change in oxygenation.

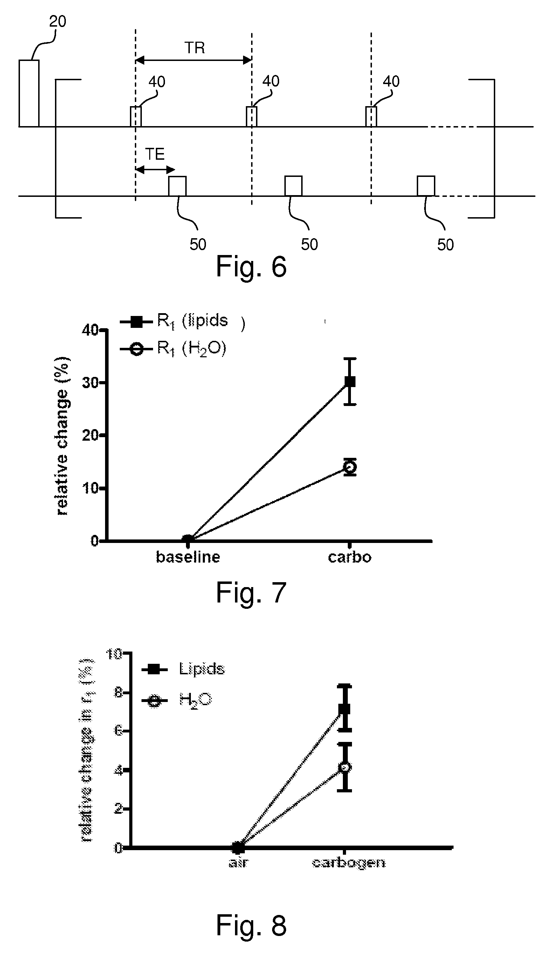

Both for figures 3 and 4, different samples were prepared following a same experimental procedure. Different liquid or semi-liquid phases (water (H20), oil, milk, liver homogenate) were mixed in a test-tube with a gas comprising a known percentage of oxygen so that to obtain an equilibrium state were the content in oxygen in the liquid or semi-liquid phase equals the content in oxygen in the gas lying above the liquid or semi-liquid phase in the test-tube. Then the different test-tubes were subjected to a static magnetic induction $B_0$ of 11.7 T, and to a sequence of RF pulses. For the samples named H20 and oil of figures 3 and 4, the inventors used a sequence of RF pulses as shown in figure 6 (no saturation pulse 30 for removing a contribution of protons of water molecules). For the sample named milk in figure 3 and liver homogenate in figure 4, a sequence of RF pulses similar to the one shown in figure 1 was used, which means a sequence of RF pulses including saturations pluses 30 for removing a contribution of protons of water molecules. Three measurements for each sample (water (H20), oil, milk, liver homogenate) were carried out corresponding to 0 % in oxygen, 21 % in oxygen and 95 % in oxygen. Then, a straight line corresponding to a linear equation was fitted between the three measurements following a least square approximation approach. From these straight lines, one can find obtain for each sample a slope of the linear equation relating the content in oxygen and the variation of proton longitudinal relaxation rate, $\Delta R1$. In figure 3, we see that the slope for the oil curve is much larger than the slope of the water curve. Hence, these measurements show that for evaluating a variation of oxygenation, a higher sensitivity can be obtained when considering a variation of proton longitudinal relaxation rate, $\Delta R1$, of lipids with respect a method that uses a variation of proton longitudinal relaxation rate, $\Delta R1$, of water molecules. Milk is a good model for evaluating a variation of proton longitudinal relaxation rate, $\Delta R1$, of mammal tissues whose oxygenation changes. Hence, the straight line with a slope of 0.013 is preferably used when determining a variation of oxygenation in a tissue 10 from a variation of proton longitudinal relaxation rate, $\Delta R1$, measured with a MRI technique. As an example, if a variation of proton longitudinal relaxation rate of lipids, $\Delta R1$, determined in vivo with the method of the invention is equal to a value that is referred as $\Delta R1(mes)$, then one can deduce that the corresponding variation of oxygenation in vivo is equal to $\Delta O2(mes) = \Delta R1$ (mes)*0.013. So, when using such an experimental calibration procedure, the abscissa of figures 3 and 4 (levels of oxygenation in in vitro conditions) are transposed into variations of oxygenation in in vivo conditions when determining a variation of oxygenation, $\Delta O2$, corresponding to a measured in vivo $\Delta R1$. The procedure of obtaining $\Delta O2(mes)$ from $\Delta R1$ (mes) by using calibration data with milk is illustrated in figure 3.

Another example of calibration medium that could be used for determining a variation of oxygenation of a mammal tissue 10 from a value of variation of proton longitudinal relaxation rate of lipids, $\Delta R1$, is a liver homogenate. The variation of $\Delta R1$ with respect to the oxygen content in a test-tube (in vitro conditions) for such a liver homogenate is shown in figure 4 (crosses).

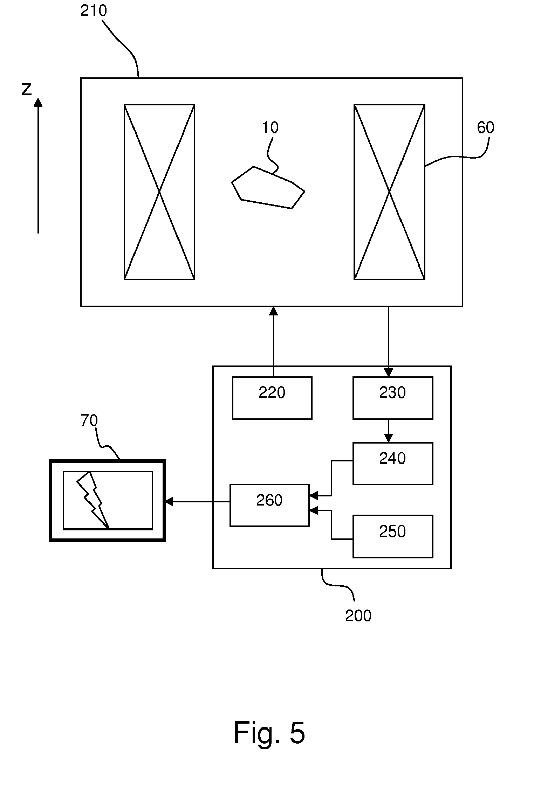

According to a second aspect, the inventors propose a device 200 able to communicate with a MRI apparatus 210. Such a device 200 is shown in figure 5 with a MRI apparatus 210. The MRI apparatus typically comprises a superconducting magnet 60 for generating a strong static magnetic induction $B_0$. The device 200 of the invention comprises a unit such as a computer 200 with a set of subunits or software modules that implement various steps of the method of the invention. The computer 200 can be an ordinary, single processor personal computer. The different software modules described below can be included in different computers or different units rather than in a single computer 200. The computer 200 also includes an internal memory (not shown in figure 5) for storing computer program instructions which control how a processing unit within the computer 200 accepts, transforms, and outputs data. The internal memory includes both a volatile and a non-volatile portion. Those skilled in the art will recognize that the internal memory can be supplemented with computer memory media, such as compact disk, flash memory cards, magnetic disc drives.

Control means 220 send instructions to the MRI apparatus 210. Such control means 220 can be a software module and the communication between the device 200 and the MRI apparatus 210 can be a serial, parallel, or Ethernet communication of any kind known by the one skilled in the art. The instructions sent by the control means 220 are such that the MRI apparatus 210 is able to measure a proton longitudinal relaxation rate R1 of lipids in a tissue 10 subjected to a static magnetic induction and a sequence of RF pulses generated by said MRI apparatus 210. Acquisition means 230 acquire at least two values of a proton longitudinal relaxation rate R1 obtained from at least two measurements carried out with said MRI apparatus on said tissue 10. Preferably, an oxygen exposure of said tissue 10 is changed between the two measurements. Then, means 240 determine a value of variation of proton longitudinal relaxation rate of lipids, $\Delta R1$, from the at least two values of R1 obtained from the at least two measurements. Means 250 provide calibration data relating values of variation of said proton longitudinal relaxation rate of lipids, $\Delta R1$, to values of variation of oxygenation. Finally, means 260 determine, by using the calibration data, a variation of oxygenation in said tissue 10 corresponding to the value of variation of said proton longitudinal relaxation rate $\Delta R1$ determined by means 240. Preferably, means 260 sends the variation of oxygenation in said tissue 10 to a display 70.

According to a third aspect, the inventors propose to use a method according to the invention for in vivo quantifying a variation of oxygenation in a tissue when said variation of oxygenation is induced by a drug. Then, the first measurement of R1 is preferably performed before an administration of the drug, and the second measurement is preferably carried out after an administration of the drug. The ability of a technique to monitor an effect of a drug on local oxygenation would be indeed very helpful in phases of development of a new drug. Pharmaceutical companies are seeking for new reliable imaging biomarkers that may demonstrate early in the clinical trials a presence or an absence of effect induced by a drug. As an example in oncology, strategies are aimed at decreasing oxygenation and perfusion of tumors by antiangiogenic therapies or the use or vascular disrupting agents. On the opposite, other strategies involve a transient reoxygenation of a tumor at the time of irradiation. This example may be extended to most ischemic diseases. In all cases, an imaging biomarker can be helpful to demonstrate non invasively that an administration of a drug induces an increase or a decrease of a tissue oxygenation (whatever the route of administration of the drug: oral, sublingual, dermal, anal or parenteral). It will also help companies to have a tool that may allow selection of patients who are more likely to benefit from a specific treatment. After registration of a drug, the same tool can also be used to reserve the treatment to patients who are likely to respond to a treatment, avoiding by this way an unnecessary treatment or side effects to patients that are not responsive to this drug-induced treatment.

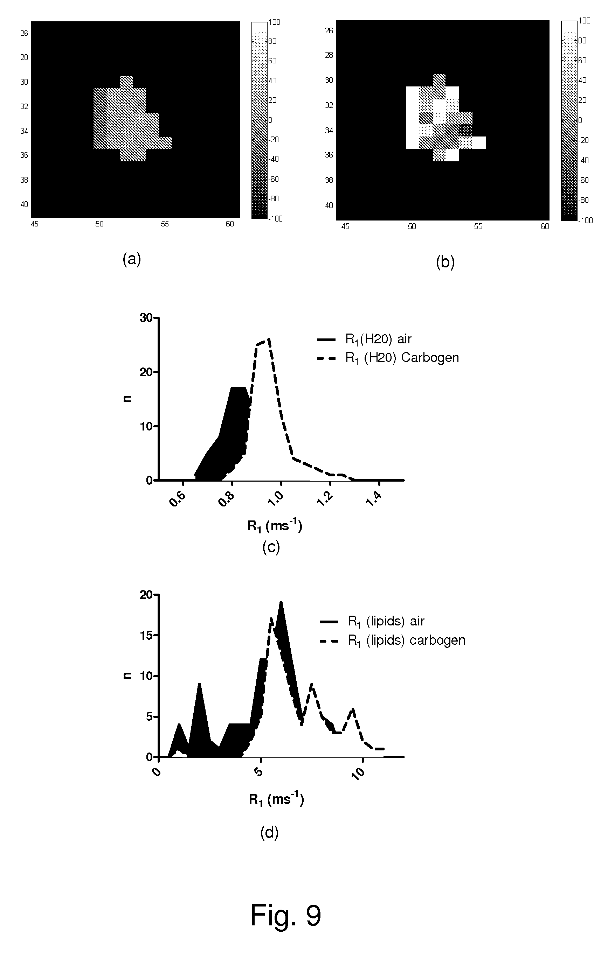

Finally some results are presented showing that a method using a variation of proton longitudinal relaxation rate, $\Delta R1$, of lipids rather than using a global $\Delta R1$ that is mainly due to water molecules, presents a higher sensitivity when determining a variation of oxygenation in tissues 10. For these results, a static magnetic induction of 11.7 T was used. When determining $\Delta R1$ of lipids, a sequence similar to the one depicted in figure 1 was used. When determining a global $\Delta R1$ the sequence depicted in figure 6 was used. Figure 7 (respectively 8) shows the relative changes of R1 of lipids and water molecules in percent in a brain (respectively liver) of a mouse. The abscissa baseline corresponds to a case where the mouse breaths normal air whereas the abscissa carbo corresponds to a case where the mouse breaths carbogen (95 % oxygen, 5 % $CO_2$). From these figures, we clearly observe that the variation $\Delta R1$ of lipids is much larger than a global $\Delta R1$ corresponding mainly to a $\Delta R1$ of water molecules. A last result is shown in figure 9. A tumor that was implanted in a mouse was studied. More particularly, proton longitudinal relaxation times T1 of said tumor where evaluated after air breathing and after a carbogen breathing. Figure 9(a) shows variations in percent of a proton longitudinal relaxation time T1 of water molecules, whereas figure 9(b) shows variations in percent of a proton longitudinal relaxation time T1 of lipids. Figure 9(c) (respectively 9(d)) shows number of pixels with given R1 values of water molecules (respectively lipids), with air and carbogen breathing. From these figures, we see that a change of oxygenation leads to higher variations of R1 of lipids.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features. Reference numerals in the claims do not limit their protective scope. Use of the verbs "to comprise", "to include", "to be composed of", or any other variant, as well as their respective conjugations, does not exclude the presence of elements other than those stated. Use of the article "a", "an" or "the" preceding an element does not exclude the presence of a plurality of such elements.

Summarized, the invention may also be described as follows. The invention relates to a device 200 and a method for quantifying a variation of oxygenation in a tissue 10 by using a magnetic resonance imaging technique. The variation of oxygenation in a tissue 10 can be quantified from a measured variation of a proton longitudinal relaxation rate $\Delta R1$ and from calibration data. The method of the invention is characterized in that the variation of proton longitudinal relaxation rate $\Delta R1$ that is used is a variation of proton longitudinal relaxation rate of lipids rather than a variation of proton longitudinal relaxation rate of water molecules.

**Claims**

1. In vivo method for quantifying a variation of oxygenation in a tissue (10) by using a magnetic resonance imaging technique comprising a generation of a static magnetic induction $B_0$ and a generation of a sequence of radio-frequency pulses, said in vivo method comprising the steps of:

   a. performing a first measurement of a proton longitudinal relaxation rate R1 with said magnetic resonance imaging technique;
   b. optionally modifying an oxygen exposure of said tissue (10);
   c. performing a second measurement of a proton longitudinal relaxation rate R1 with said magnetic resonance imaging technique;
   d. determining a value of variation of said proton longitudinal relaxation rate, $\Delta R1$, from the measurements carried out in steps a. and c.;
   e. providing calibration data relating values of variation of said proton longitudinal relaxation rate, $\Delta R1$, to values of variation of oxygenation;
   f. determining a value of variation of oxygenation in said tissue (10) by using the value of variation of said proton longitudinal relaxation rate determined in step d., $\Delta R1$, and the calibration data provided in step e.;

   and **characterized in that**

- said proton longitudinal relaxation rate R1 is a proton longitudinal relaxation rate R1 of lipids.

2. Method according to claim 1 **characterized in that** said sequence of radio-frequency pulses is able to remove a contribution of protons of water molecules to said proton longitudinal relaxation rate R1 of lipids.

3. Method according to claim 2 **characterized in that** said sequence of radio-frequency pulses comprises at least one saturation pulse (30) able to predominantly excite protons of water molecules.

4. Method according to claim 3 **characterized in that** said sequence of radio-frequency pulses further comprises at least one initial inversion pulse (20), and at least one excitation pulse (40) of flip angle $\alpha$.

5. Method according to claim 4 **characterized in that** said flip angle $\alpha$ has a value equal to or smaller than 30°.

6. Method according to claim 4 or 5 **characterized in that** said at least one excitation pulse (40) has a mean frequency and it that said mean frequency is shifted by a frequency shift below a resonance frequency of water molecules.

7. Method according to claim 6 **characterized in that** said frequency shift is comprised between 0.8 ppm and 1 ppm of said resonance frequency of water molecules.

8. Method according to claim 6 **characterized in that** said frequency shift is comprised between 3.3 ppm and 3.7 ppm of said resonance frequency of water molecules.

9. Method according to any of previous claims **characterized in that** said calibration data are obtained from an in vitro experimental calibration procedure where levels of oxygenation from said in vitro experimental calibration procedure are transposed into values of variation of oxygenation.

10. Method according to any of previous claims **characterized in that** the optional step b. comprises an inhalation of an oxygen-enriched gas by a mammal body comprising said tissue (10).

11. Method according to any of previous claims **characterized in that** the optional step b. comprises a placement of a tourniquet that allows a stricture of a part of a mammal body comprising said tissue (10).

12. Device (200) able to communicate with a magnetic resonance imaging apparatus (210) and comprising:

    i. control means (220) for sending instructions to said magnetic resonance imaging apparatus (210);
    ii. acquisition means (230) for acquiring at least two values of a proton longitudinal relaxation rate R1 obtained from at least two measurements carried out with said magnetic resonance imaging apparatus (210) on a tissue (10);
    iii. means (240) for determining a value of variation of said proton longitudinal relaxation rate, $\Delta$R1, from the at least two values of a proton longitudinal relaxation rate R1 obtained from the at least two measurements;
    iv. means (250) for providing calibration data relating values of variation of said proton longitudinal relaxation rate $\Delta$R1 to values of variation of oxygenation;
    v. means (260) for determining, by using the calibration data, a variation of oxygenation in said tissue (10) corresponding to the value of variation of said proton longitudinal relaxation rate $\Delta$R1 determined by means (240);

    **characterized in that**
    said instructions sent by said control means (220) to said magnetic resonance imaging apparatus (210) are such that said magnetic resonance imaging apparatus (210) is able to measure a proton longitudinal relaxation rate R1 of lipids.

13. Use of a method according to any of claims 1 to 9 for in vivo quantifying a variation of oxygenation in a tissue (10) induced by a drug.

Fig. 1

Fig. 2

Fig. 3

Fig. 4

Fig. 5

Fig. 6

Fig. 7

Fig. 8

## Fig. 9

Europäisches
Patentamt

European
Patent Office

Office européen
des brevets

**EUROPEAN SEARCH REPORT**

Application Number

EP 11 16 3573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/070410 A1 (MEDI PHYSICS INC [US]; DRIEHUYS BASTIAAN [US]; HALL MARGARET [GB]; MAR) 19 August 2004 (2004-08-19) <br> * page 21, line 12 - line 19 * <br> * page 22, line 14 - page 23, line 12 * <br> * page 24, line 1 - line 15 * <br> * page 25, line 1 - line 13; figure 5 * | 1,4,5, 12,13 | INV. <br> A61B5/055 <br> G01R33/465 |
| A | D. GRUCKER: "Oxymetry by magnetic resonance: applications to animal biology and medicine", <br> PROGRESS IN NUCLEAR MAGNETIC RESONANCE SPECTROSCOPY, <br> vol. 36, no. 3, 1 May 2000 (2000-05-01), pages 241-270, XP55008612, <br> ISSN: 0079-6565, DOI: <br> 10.1016/S0079-6565(99)00022-9 <br> * page 246, paragraph 3.1.1.1 * | 1,12 | |
| A,D | O'CONNOR J P B ET AL: "Preliminary Study of Oxygen-Enhanced Longitudinal Relaxation in MRI: A Potential Novel Biomarker of Oxygenation Changes in Solid Tumors", <br> INTERNATIONAL JOURNAL OF RADIATION: ONCOLOGY BIOLOGY PHYSICS, PERGAMON PRESS, USA, <br> vol. 75, no. 4, <br> 15 November 2009 (2009-11-15), pages 1209-1215, XP026708263, <br> ISSN: 0360-3016, DOI: <br> 10.1016/J.IJROBP.2008.12.040 <br> [retrieved on 2009-03-26] <br> * the whole document * | 1,12,13 | TECHNICAL FIELDS SEARCHED (IPC) <br><br> A61B <br> G01R |

-/--

The present search report has been drawn up for all claims

| Place of search | Date of completion of the search | Examiner |
|---|---|---|
| The Hague | 11 October 2011 | Hunt, Brynley |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 2 514 361 A1**

| | Europäisches Patentamt European Patent Office Office européen des brevets | **EUROPEAN SEARCH REPORT** | **Application Number** EP 11 16 3573 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROBINSON ET AL: "Noninvasive Monitoring of Carbogen-Induced Changes In Tumor Blood Flow and Oxygenation by Functional Magnetic Resonance Imaging", INTERNATIONAL JOURNAL OF RADIATION: ONCOLOGY BIOLOGY PHYSICS, PERGAMON PRESS, USA, vol. 33, no. 4, 1 January 1995 (1995-01-01), pages 855-859, XP002097897, ISSN: 0360-3016, DOI: 10.1016/0360-3016(95)00072-1 * the whole document * | 1,12,13 | |
| A | US 6 307 368 B1 (VASANAWALA SHREYAS S [US] ET AL) 23 October 2001 (2001-10-23) * the whole document * | 1,12,13 | |
| A | US 2008/272782 A1 (LIN FA-HSUAN [US]) 6 November 2008 (2008-11-06) * the whole document * | 1,12,13 | |
| A | WO 2011/027165 A1 (GREATER GLASGOW HEALTH BOARD [GB]; UNIV GLASGOW [GB]; SANTOSH CELESTIN) 10 March 2011 (2011-03-10) * the whole document * | 1,12,13 | TECHNICAL FIELDS SEARCHED (IPC) |

The present search report has been drawn up for all claims

| Place of search | Date of completion of the search | Examiner |
|---|---|---|
| The Hague | 11 October 2011 | Hunt, Brynley |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 16 3573

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2011

| Patent document cited in search report | | Publication date | Patent family member(s) | | Publication date |
|---|---|---|---|---|---|
| WO 2004070410 | A1 | 19-08-2004 | AU | 2004209434 A1 | 19-08-2004 |
| | | | AU | 2010202158 A1 | 24-06-2010 |
| | | | CA | 2512382 A1 | 19-08-2004 |
| | | | CN | 1745314 A | 08-03-2006 |
| | | | EP | 1588180 A1 | 26-10-2005 |
| | | | JP | 2006516460 A | 06-07-2006 |
| | | | KR | 20050099513 A | 13-10-2005 |
| | | | US | 2007110669 A1 | 17-05-2007 |
| | | | US | 2011104067 A1 | 05-05-2011 |
| US 6307368 | B1 | 23-10-2001 | NONE | | |
| US 2008272782 | A1 | 06-11-2008 | NONE | | |
| WO 2011027165 | A1 | 10-03-2011 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M HOCKEL et al.** *J Natl Cancer Inst,* 2001, vol. 93, 266-276
- **J VON PAWEL et al.** *J Clin Oncol,* 2000, vol. 18, 1351-1359
- **G KARCZMAR.** *NMR Biomed,* 1994, vol. 7, 3-11
- **S ROBINSON.** *Int J Radiat Oncol Biol Phys,* 1995, vol. 33, 855-859
- **C BAUDELET.** *Magn Reson Med,* 2002, vol. 48, 980-986
- *Curr. Med. Imag. Reviews,* 2005, vol. 1, 229-243
- **JORDAN.** *Magn. Reson Med,* 2006, vol. 56, 637-643

- **J P B O'CONNOR.** *Int J Radiation Oncology Biol Phys,* 2009, vol. 75 (4), 1209-1215
- **KODIBAGKAR VD.** *NMR Biomed,* 2008, vol. 21, 899-907
- **RD BRAUN.** *Am J Physiol,* 1999, vol. 277, H551-568
- **HAACKE.** Magnetic Resonance Imaging: Physical Principles and Sequence Design. John Willey & sons, 1999
- Quantitative MRI of the brain. **GOWLAND ; STEVENSON.** The longitudinal relaxation time. 2004, 111-141
- **M KRIAT et al.** *Journal of Lipid Research,* 1998, vol. 34, 1009